# EUROPEAN PATENT APPLICATION

(11) **EP 3 424 550 A1**
(43) Date of publication of application: **09.01.2019**
(21) Application number: 17305878.5
(22) Date of filing: 06.07.2017
(51) Int. Cl.: A61M 16/12, A61K 9/00, A61K 33/00

(54) **GAS MIXTURES CONTAINING LOW CONCENTRATIONS OF XENON AND ARGON PROVIDE NEUROPROTECTION WITHOUT INHIBITING THE CATALYTIC ACTIVITY OF THROMBOLYTIC AGENTS**

(71) Applicant: Monatomics Technology, 75016 Paris (FR)
(72) Inventor: DAVID, Hélène, Québec G0A 3Z0 (CA)
(74) Representative: Ex Materia

(57) **Abstract**

The present invention relates to low concentration synergistic Xenon-Argon gas mixtures argon for use in a method for preventing and/or treating ischemic insults, wherein
- the volume proportion of xenon is between 5 and 20 %,
- the volume proportion of argon is between 5 and 20 %, and
- the pharmaceutical composition further comprises a gas complement to reach 100 % volume proportion,
- the method includes comprises restoration of blood flow and the gas mixture is administered to the patient before, during, or after blood flow restoration.

## Description

### Technical Field

The present invention relates to the field of organ protection, in particular neuroprotection, and gas mixtures for use as organ-protective, particularly neuroprotective, agents, in the treatment and/or prevention of ischemic insults.

### Technological Background

Ischemia is a restriction in blood supply generally due to factors in the blood vessels, particularly thromboembolism (blood clots), which lead to tissue dysfunction and cell death through necrotic and apoptotic mechanisms. Ischemia is an absolute or relative shortage of the blood supply to an organ. Relative shortage means the mismatch of blood supply and blood request for adequate oxygen (and glucose) delivery in tissue. The extent of tissue damage mainly depends on the level and duration of ischemia. The heart, the kidneys, and the brain are among the organs that are the most sensitive to inadequate blood supply. For instance, ischemic stroke (also called brain attack or acute cerebral ischemia) and myocardial infarction (also called heart attack or acute cardiac ischemia) are with cancer the major causes of death in humans. It is estimated that global cardiovascular deaths will increase from 17 million deaths to more than 23 million deaths in 2030, and that cerebral stroke will represent more than 6 % of the diseases' global impact in 2020-2025 with nearly 25 % of males and 20% of females who will suffer a brain attack before reaching 85-year old.

Proteolysis is a general catalytic physiological process, which can be defined as the directed (oriented) degradation of proteins by cellular enzymes called proteases. Fibrinolysis is a specific case of proteolysis. Fibrinolysis is the physiological process wherein a fibrin clot, the product of coagulation, is broken down. In the case of vascular injury, such as the production of blood (fibrin) clot, endothelial cells release a serine protease called tissue-type plasminogen activator (tPA) that converts the proenzyme plasminogen to plasmin, the main enzyme of fibrin, which cuts the fibrin mesh. In healthy subjects, this process allows avoiding excessive clot formation and ischemic accidents. In patients suffering from thromboembolism and ischemia, fibrinolysis can be stimulated through administration of the human recombinant form of tissue-type plasminogen activator (rtPA). This breakdown of blood clots by pharmacological means is called thrombolysis. Thrombolysis is the major therapeutic strategy for treating ischemic insults. However, under certain conditions, thrombolytic therapy is associated with a risk of hemorrhagic transformation and neuronal death potentiation that is due to the general proteolytic properties of plasmin. To avoid such adverse side effects of plasmin, rtPA has to be administered to the patient within an appropriate period, called "therapeutic window", which is typically of up to 3 to 4.5 hours after ischemia onset according to current medical practice and knowledge.

Acute cerebral ischemia is caused by a reduction of blood flow in the brain. This leads more or less to brain dysfunctions and damage and neuronal death. The extent of brain injury mainly depends on the level and duration of ischemia. The physiological processes involved in ischemia-induced neuronal death are complex. Briefly, the reduction in cerebral blood flow compromises tissue energy stores and leads to a deficit in oxygen and glucose. At the cellular level, a critical consequence of this metabolic deprivation is an increase of the intracellular sodium concentration. This leads to an exaggerated efflux and uptake failure of many neurotransmitters, among which is glutamate [14]. The excessive release of glutamate over-activates N-methyl-D-aspartate (NMDA) receptors. This results in a NMDA receptor-mediated neuronal depolarization and intraneuronal calcium influx that overstep the physiological bounds and lead to neuronal death through necrotic and apoptotic mechanisms [15,16]. Theref-ore, two strategies have been pursued for the treatment of ischemic stroke: a limitation of the vascular insult by early reperfusion and/or a blockade of the neurotoxic cascade initiated by glutamate.

Today, early reperfusion by rtPA-induced thrombolysis is the only pharmaceutical treatment of stroke approved by the Food and Drug Administration and the European Medical Agencies. However, as stated above, despite its benefial effects, thrombolytic therapy is associated with a risk of hemorrhagic transformation and neuronal death potentiation [17,18].

Patent US 8,435,569 thus proposed a method for treating and/or preventing ischemic insults combining thrombolysis and neuroprotective gases such as nitrous oxide, xenon, argon, helium, neon, and mixtures thereof. Particularly, Patent US 8,435,569 discloses the use of at least one thrombolytic agent (A), such as rtPA, and at least one gas (B) selected from the group consisting of nitrous oxide, argon, xenon, helium, neon, and mixtures thereof, for the preparation of a combined pharmaceutical composition for treating ischemia. More particularly, since nitrous oxide, argon, xenon, helium, neon, and mixtures thereof, interact with rtPA, Patent US 8,435,569 discloses how these neuroprotective gases should be administered before, together with, or after the thrombolytic agent (A) in order not to block or reduce the beneficial thrombolytic effects of the thrombolytic agent (A). However, Patent US 8,435,569 does not disclose any gas or gas mixtures, which would be cost-efficient, highly therapeutically efficient, and would not interact with thrombolytic agent (A), such as rtPA.

Previous studies in models of hypoxic-ischemic brain insults have demonstrated the neuroprotective potential and lack of toxic effects of the chemically and metabolically inert gases xenon and argon [1-7].

Among the inert and noble gases and other molecules, xenon at concentrations above 35-50 vol% is considered the gold standard due to its potent organprotective and neuroprotective effects. Unfortunately, xenon is also a potent inhibitor of tPA and rtPA, a condition that precludes its use before or together with rtPA due to the risk of blocking the beneficial thrombolytic effects of tPA and rtPA [10]. In addition, the excessive cost of production further limits its widespread clinical use.

In contrast, argon is a cost-efficient gas that shows no inhibiting action on the beneficial thrombolytic properties of tPA and rtPA when used at efficient neuroprotective concentrations of 50 vol% and above [11]. Unfortunately, argon is far less potent than xenon at providing neuroprotection in models of acute ischemic stroke [3,6].

To date, there thus remains a need for a cost-efficient organ protective, in particular neuroprotective agent, which may in particular be used before, together with, or after administration of a thrombolytic agent, advantageously in any order of administration.

### Summary of the invention

Surprisingly, the Inventors discovered that gas mixtures containing low concentrations of xenon and argon provides neuroprotection without inhibiting thrombolysis, thus allowing these gas mixtures to be used before, together with, or after administration of thrombolytic agents. Without wishing to be bound by theory, it is hypothesized that the synergistic neuroprotective effects of xenon and argon at low concentrations arise from their complementary mode of action: while xenon is thought to provide neuroprotection mainly through antagonism at the glutamatergic N-methyl-D-aspartate receptor (NMDA) [8], which is the main excitatory receptor in the brain, argon is believed to act mainly by potentiating the γ-aminobutyric acid type A (GABA-A) [9] receptor that is the main inhibitory receptor in the brain.
The inventors indeed investigated, using *in vitro, ex vivo,* and *in vivo* preparations, the effects of gas mixtures containing equimolar concentrations of xenon (Xe) and argon (Ar) of 15 (Xe-Ar-15), 25 (Xe-Ar-25) and 37.5 vol% (Xe-Ar-37.5) on cell injury induced by oxygen and glucose deprivation (OGD) in brain slices, brain damage in rats subjected to an intracerebral injection of NMDA, and the catalytic efficiency of rtPA, the basic mechanism by which rtPA acts and thrombolysis occurs. Particularly, they found Xe-Ar-15 > Xe-Ar-25 > Xe-Ar-37.5 at providing neuroprotection, and Xe-Ar-15 < Xe-Ar-25 < Xe-Ar-37.5 at reducing the catalytic activity of rtPA. Interestingly, Xe-Ar-15 provides neuroprotection as potent as xenon alone at 50 vol% and above but, unlike xenon, shows no effect on the catalytic and thrombolytic activity of rtPA.

Therefore, in a first aspect, the present invention relates to a pharmaceutical composition comprising a gas mixture of xenon and argon for use in a method for preventing and/or treating ischemic insults, wherein
- the volume proportion of xenon is between 5 and 20 %,
- the volume proportion of argon is between 5 and 20 %, and
- the pharmaceutical composition further comprises a gas complement to reach 100 % volume proportion,
- the method further includes (spontaneous or medical) restoration of blood flow and administering said pharmaceutical composition to the patient before, during, or after blood flow restoration.

In a second aspect, the present invention relates to the use of a pharmaceutical composition comprising a gas mixture of xenon and argon, wherein
- the volume proportion of xenon is between 5 and 20 %,
- the volume proportion of argon is between 5 and 20 %, and
- the pharmaceutical composition further comprises a gas complement to reach 100 % volume proportion,
to manufacture a medicament for preventing and/or treating ischemic insults, wherein the method further includes (spontaneous or medical) restoration of blood flow, and administering said pharmaceutical composition to the patient before, during, or after blood flow restoration. In a third aspect, the present invention relates to a method for preventing and/or treating ischemic insults, comprising administering to a patient in need thereof an effective dose of a pharmaceutical composition comprising a gas mixture of xenon and argon, wherein
- the volume proportion of xenon is between 5 and 20 %,
- the volume proportion of argon is between 5 and 20 %, and
- the pharmaceutical composition further comprises a gas complement to reach 100 % volume proportion,
wherein the method further includes (spontaneous or medical) restoration of blood flow and administering said pharmaceutical composition to the patient before, during, or after blood flow restoration.

In the present invention, the "patient" is in particular an animal such as a mammal, and preferably a human. The present invention has thus applications in both human and veterinary medicine.
In the method for preventing and/or treating ischemic insults, the gas mixture of xenon and argon is used as an organ protectant, in particular as neuroprotectant, meaning that it provides organ protection, more specifically neuroprotection.
In the present invention, "organ protection" is understood as the relative preservation of the cellular structure and function of an organ which has become dysfunctional due to a primary or secondary, direct or indirect, insult. Providing organ protection thus aims at treating and/or slowing and/or preventing disease progression by stopping or at least slowing organ cell death. In the present invention, "neuroprotection" is a specific type of organ protection, understood as the relative preservation of the neuronal structure and/or function. Providing neuroprotection thus aims at treating and/or slowing and/or preventing disease progression and secondary injuries by stopping or at least slowing neuronal death (or loss of neurons).
As used herein, "medical" blood flow restoration means that blood flow is restored by human intervention and not naturally. In contrast, spontaneous blood flow restoration occurs naturally, without any human intervention, in particular without administering any pharmaceutical (thrombolytic) agent.
As used herein, the "volume proportion" (vol%) of a gas is a percentage calculated on the basis of the volume of the gaseous composition as a whole. Where appropriate, the volume proportion is based on the volume of the decompressed, reconstituted, gaseous composition as a whole. In the following, the "concentration of a gas" is understood as the volume proportion of said gas.

### Detailed Description

The present invention concerns a pharmaceutical composition comprising a gas mixture of xenon and argon for use in a method for preventing and/or treating ischemic insults, wherein
- the volume proportion of xenon is between 5 and 20 %,
- the volume proportion of argon is between 5 and 20 %, and
- the pharmaceutical composition further comprises a gas complement to reach 100 % volume proportion,
- the method further comprises restoration of blood flow and administering the pharmaceutical composition to the patient before, during, or after blood flow restoration. Advantageously, the xenon/argon volume ratio is between 4/1 and 1/4, preferably between 3/1 and 1/3, more preferably between 2/1 and 1/2, most preferably it is of 1/1 (*i.e.* equimolar mixtures).
In a particular embodiment, the pharmaceutical composition comprises a gas mixture of xenon and argon wherein:
- the volume proportion of xenon is between 10 and 20 %, and
- the volume proportion of argon is between 10 and 20%. In this embodiment, the gas mixture is advantageously equimolar.
In a particular example, the volume proportion of xenon is 15%, and the volume proportion of argon is 15%.

The pharmaceutical composition of the invention is intended for any appropriate route of administration, preferably through inhalation. The synergistic composition of the invention is thus preferably inhalable. It thus comprises a gas complement. Typically, the gas complement comprises oxygen, nitrogen, helium, hydrogen, neon, or a mixture thereof. Of course, only stable, non-explosive gas mixtures are contemplated. In particular, in the present invention, when the gas complement comprises hydrogen and oxygen, these gases are in stable, non-explosive proportions.
Preferably, the gas complement comprises oxygen, typically in a volume proportion of between 20% and 50% (such as between 25% and 45% or between 30% and 40%, for instance the volume proportion of oxygen is 20%, 25%, 30%, 35%, 40%, 45% or 50%), and the remainder of the gas in the gas complement is advantageously nitrogen, helium, hydrogen, neon or a mixture thereof, preferably the remainder of the gas in the gas complement is advantageously nitrogen, helium, neon or a mixture thereof, even more preferably it is helium. Most preferably, the gas complement comprises oxygen typically in a volume proportion of between 20% and 50%, and the remainder of the gas in the gas complement is helium.
In a first embodiment, the pharmaceutical composition is preconditioned as a compressed gas mixture, and administered to the patient as a decompressed gas mixture.
In a second embodiment, the pharmaceutical composition administered to the patient is obtained by mixing argon, xenon and the gas complement in a gas mixer (and administered to the patient as a mixture of decompressed gas). In this second embodiment, argon, xenon and the gas complement are preconditioned as compressed gas in individual pressurized compartments, and decompressed prior to or upon mixing. Of note, the gas complement(s) may itself be a gas mixture (typically comprising oxygen), which may also be obtained by mixing different gases into the gas mixer. Therefore, in this second embodiment, argon, xenon, oxygen and optionally other individualized gas tanks are connected to the gas mixer, wherein they are mixed in the desired volume proportions upon or after decompression. Preferably, each gas is decompressed prior to being introduced into the gas mixer.

Typically, the ischemic insult is cerebral ischemia, cardiac ischemia, renal ischemia, retinal ischemia, or lower limb's ischemia. It may also be caused by a decompression accident (also known as decompression sickness or decompression illness).
The "effective dose" of the composition of the invention, comprising a synergistic mixture of xenon and argon, varies as a function of numerous parameters such as, for example, the route of administration and the weight, the age, the sex, the advancement of the pathology to be treated and the sensitivity of the subject or patient to be treated.
Blood flow may be restored by different methods, which are selected by the physician according to the cause of the ischemic insult, and may also depend from numerous parameters such as, for example, the weight, the age, the sex, the advancement of the pathology to be treated and the sensitivity of the subject or patient to be treated.
In a particular embodiment, the synergistic xenon-argon gaseous composition of the invention is administered before blood flow restoration, especially when blood flow is restored by thrombolysis induction.

In a particular embodiment, the synergistic xenon-argon gaseous composition of the invention is administered after blood flow restoration.
In a particular embodiment, the synergistic xenon-argon gaseous composition of the invention is administered during blood flow restoration, especially when blood flow is restored by thrombolysis induction.
Blood flow may be restored spontaneously or medically.
Typically, in case of medical blood flow restoration, blood flow is restored by thrombolysis and/or by thrombectomy.
Thrombolysis may be induced by administering one or more than one pharmaceutical agents with thrombolysis properties such as thrombolytic agents, for example streptokinase, urokinase, alteplase (human recombinant tissue-type plasminogen activator or rtPA), reteplase or tenecteplase. As used herein, the term "pharmaceutical agents with thrombolysis properties" is understood as a pharmaceutical compound initially developed as a thrombolytic agent or as a drug showing thrombolytic properties, even if initially developed for other medical use, such as N-acetylcysteine or N-Acetyl-L-cysteine for instance. A preferred thrombolytic agent is rtPA.
Thrombectomy is a well-known surgical procedure allowing removal of blood clot(s).
In an advantageous embodiment, blood flow is restored by thrombectomy, preferably with concomitant thrombolysis. In the case of both thrombolysis and thrombectomy are performed, the patient is administered a pharmaceutical agent with thrombolysis properties such as a thrombolytic agent, for example streptokinase, urokinase, alteplase (human recombinant tissue-type plasminogen activator or rtPA), reteplase or tenecteplase (preferably rtPA). Thrombectomy is typically performed before or after the patient is given the thrombolytic agent, preferably after.
Under certain circumstances, the ischemic insult is caused by a decompression accident. In such a case, blood flow is advantageously restored by thrombolysis and/or by thrombectomy and/or recompression. Preferably, in such a case, blood flow restoration comprises or consists of recompression.
In a particular embodiment of the invention (no matter what ischemic insult is concerned), blood flow restoration does not comprise administering a pharmaceutical agent with thrombolysis properties such as a thrombolytic agent.

### Description of the figures

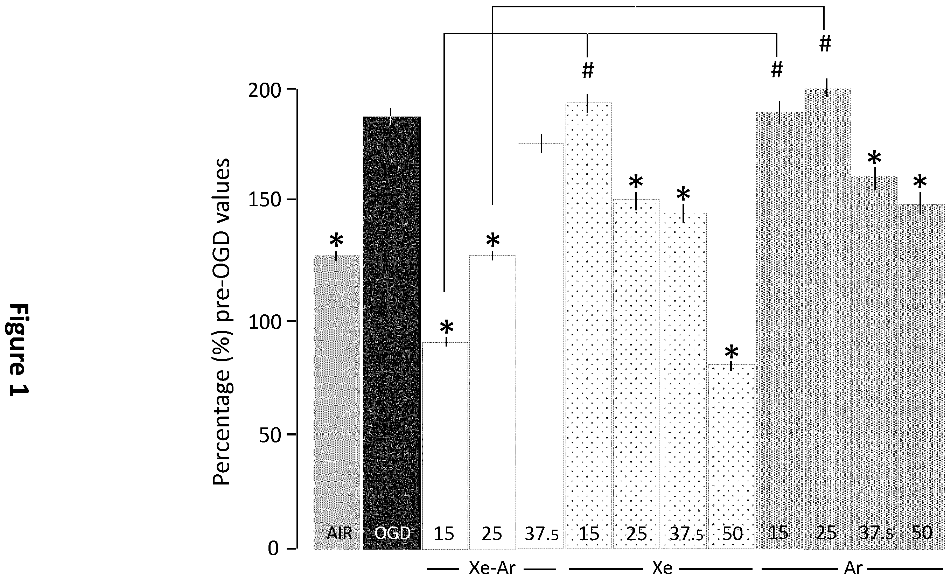

Figure 1. Effects of gas mixtures containing equimolar concentrations of xenon and argon of 15 vol% to 37.5 vol% on the release of lactate dehydrogenase (LDH) induced by oxygen-glucose deprivation (OGD) expressed as a percentage of pre-OGD values. Xe-Ar-15: gas mixture with equimolar concentration of xenon and argon of 15 vol%; Xe-Ar-25: gas mixture with equimolar concentration of xenon and argon of 25 vol%; Xe-Ar-37.5: gas mixture with equimolar concentration of xenon and argon of 37.5 vol%. Xe-Ar-15 approximately reduced OGD-induced LDH release in a manner similar to that of xenon at 50 vol%. Significant differences are marked by stars and sharp. **P* < 0.0001 *vs* OGD slices; # P < 0.0001 vs xenon or argon alone at 15 vol% or 25 vol%.

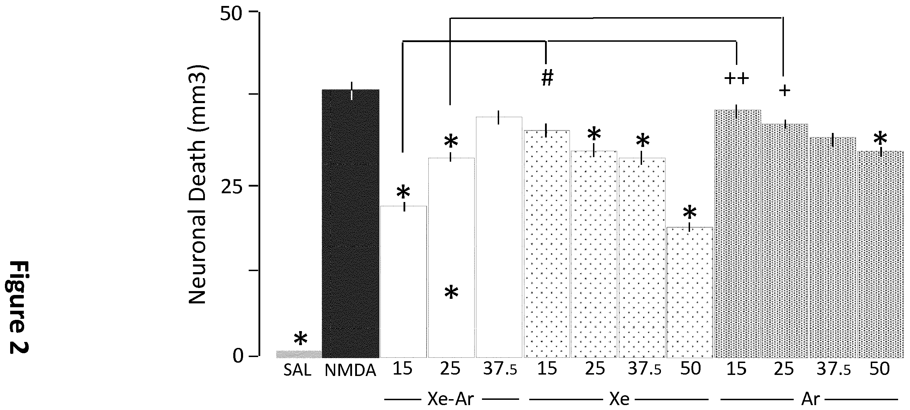

Figure 2. Effects of gas mixtures containing equimolar concentrations of xenon and argon of 15 vol% to 37.5 vol% on brain damage induced by intracerebral injection of N-methyl-D-aspartate (NMDA) expressed in mm³. Xe-Ar-15: gas mixture with equimolar concentration of xenon and argon of 15 vol%; Xe-Ar-25: gas mixture with equimolar concentration of xenon and argon of 25 vol%; Xe-Ar-37.5: gas mixture with equimolar concentration of xenon and argon of 37.5 vol%. Xe-Ar-15 approximately reduced NMDA-induced brain damage in a manner similar to that of xenon at 50 vol%. Significant differences are marked by stars, sharps, or plus symbols. ** P* < 0.0001 *vs* OGD slices; # *P* < 0.002 vs xenon at 15 vol%; ⁺⁺ *P* < 0.02 vs argon at 15 vol%; ⁺ *P* < 0.05 vs argon at 25 vol%.

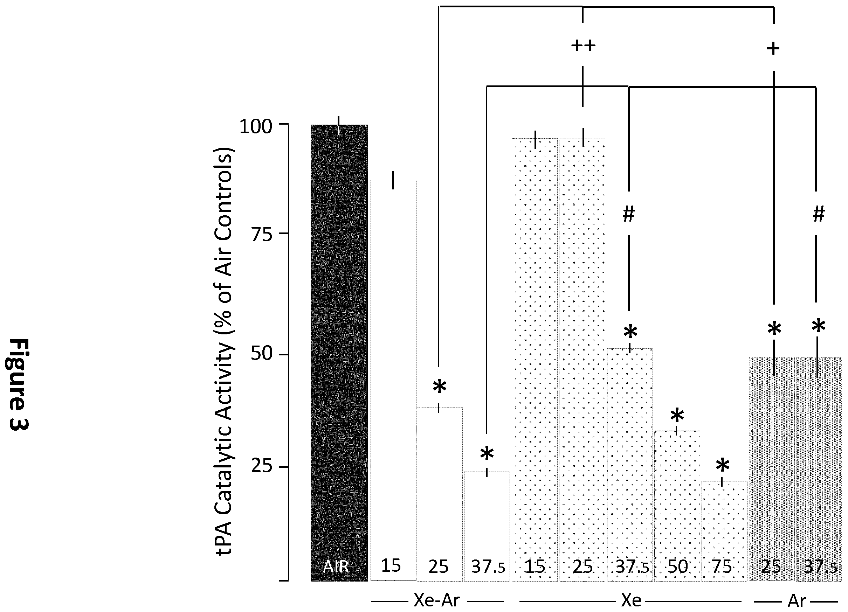

Figure 3. Effects of gas mixtures containing equimolar concentrations of xenon and argon of 15 vol % to 37.5 vol % on the catalytic efficiency of tissue plasminogen activator as expressed in percentage of air controls values. Xe-Ar-15: gas mixture with equimolar concentration of xenon and argon of 15 vol%; Xe-Ar-25: gas mixture with equimolar concentration of xenon and argon of 25 vol%; Xe-Ar-37.5: gas mixture with equimolar concentration of xenon and argon of 37.5 vol%. Xe-Ar-15, unlike Xe-Ar-25 and Xe-Ar-37.5, does not reduce the catalytic efficiency of rtPA. Significant differences are marked by star, sharp or plus symbol. * *P* < 0.0001 *vs* OGD slices; ⁺⁺ *P* < 0.0001 vs xenon at 25 vol%; ⁺ *P <* 0.002 vs xenon at 25 vol%. # P < 0.0001 vs xenon at 37.5 vol% and argon at 37.5 vol%.

### EXAMPLES

The present invention is illustrated by the following examples, which are not to be construed as limiting the invention in any way.

### MATERIALS AND METHODS

### Animals

All animal-use procedures were performed in accordance with the Declaration of Helsinki and were within the framework of the French legislation for the use of animals in biomedical experimentation. Adult male Sprague-Dawley rats (Janvier, Le Genest Saint-Isle, France) weighing 250-280 g were used. Before being used, rats were housed at 21 ± 0.5°C in Perspex home cages with free access to food and water. Light was maintained on a light/dark reverse cycle, with lights on from 8:00 PM to 8:00 AM.

### Preparation and incubation of brain slices

Rats were killed by decapitation under halothane anesthesia. The brains were removed and placed in ice-cold freshly prepared artificial cerebrospinal fluid (aCSF). Coronal brain slices (400 µm thickness) including the striatum (anteriority: from +1.2 to +2 mm from bregma) were cut using a tissue chopper (Mickie Laboratory Engineering Co., Gomshall, Surrey, UK).

### Measurement of cell injury with lactate dehydrogenase activity assay

The effects of gas mixtures containing xenon and argon on the release of lactate dehydrogenase (LDH) induced by OGD were assessed as described previously [6]. Before being used, brain slices were transferred into individual vials with 1.3 ml of freshly prepared oxygenated aCSF containing 120 mM NaCl, 2 mM KCI, 2 mM CaCl₂, 26 mM NaHCO₃, 1.19 mM MgSO₄, 1.18 mM KH₂PO₄, 11 mM d-glucose, and 30 mM HEPES and allowed to recover at room temperature for 45 min. Then, brain slices were placed at 36 ± 0.5°C into individual vials containing 1.3 ml of freshly prepared aCSF, saturated, and continuously bubbled with 100 % oxygen (25 ml/min per vial). After a 30-min period, the incubation aCSF solution was renewed with oxygenated aCSF maintained at 36°C, and the slices were then incubated for 1 h to allow recording of basal levels of LDH. Whereas control slices were incubated for an additional 20-min period in the same conditions, those corresponding to the ischemic group were incubated in a glucose-free solution, saturated, and continuously bubbled with 100 % nitrogen (OGD slices). After this 20-min period of OGD, to mimic reperfusion and treatment, the medium was replaced in all groups with freshly prepared aCSF solution, saturated and continuously bubbled with either medical air or gas mixtures containing xenon and argon in volume proportions as described below (see gas pharmacology section; n = 20-36).

### NMDA-induced neuronal death in vivo

The effects of of gas mixtures containing xenon and argon on the catalytic activity of tPA were assessed as described previously [6]. On the day of surgery, rats were anesthetized with 1.5% halothane in oxygen alone in an anesthesia box and mounted on a stereotactic apparatus with the incisor bar set at 3.9 mm below the horizontal zero. A burr hole was drilled and a micropipette (less than 10 µm at the tip) was lowered into the right striatum (anterior, 0.6 mm; lateral, 3.0 mm; ventral, 5.8 mm, from bregma) to allow injection of 70 nmol of NMDA in 1 µl of phospahte buffered solution (pH 7.4) over a 2-min period. After an additional 5-min period, the micropipette was removed, and the wounds were then sutured. During surgery, body temperature was kept at 37 ± 0.5°C with a feedback-controlled thermostatic heating pad (Harvard Apparatus Limited, Edenbridge, UK). The animals woke up in their home cage after about 10 min, where they were given free access to food and water. Sixty minutes after NMDA administration, rats were treated with medical air or gas mixtures containing xenon and argon (see gas pharmacological section). The number of rats per group was n = 7-11.

### In vitro tPA Catalytic Activity Assay

The effects of gas mixtures containing xenon and argon on the catalytic activity of tPA were assessed as described previously [6]. The recombinant form of human tPA (Actilyse®; Boehringer Ingelheim, Ingelheim am Rhein, Germany) and its specific chromogenic substrate methylsulfonyl-D-phenyl-glycil-arginine-7-amino-4-methylcoumarin acetate (Spectrozyme® XF, product 444; American Diagnostica, Stamford, CT) were diluted separately in 1 ml distilled water in 1.5-ml sterile tubes. Each tube containing 0.4 µM tPA or 10 µM tPA substrate was saturated for 20 min at a flow rate of 60-80 ml/min with medical air (controls) or gas mixtures containing xenon and argon in volume proportions as described below (n = 9-14 per concentration). The catalytic efficiency of tPA was assessed by the initial rate method by incubating 50 µl tPA with 50 µl substrate in a spectrofluorometer microplate reader set at 37 °C.

### Gas Pharmacology

Xenon, argon, nitrogen, and oxygen were purchased from Air Liquide Santé (Paris, France). Medical air composed of 75 vol% nitrogen and 25 vol% oxygen, and xenon and argon mixtures containing 37.5 vol% xenon and 37.5 vol% argon (Xe-Ar-37.5), 25 vol% xenon and 25 vol% argon (Xe-Ar-25) or 15 vol% xenon and 15 vol% argon (Xe-Ar-15) - with 25 vol% oxygen and the remainder being nitrogen when needed - were obtained using computer-driven gas mass flowmeters, and an oxygen analyzer to check that the gas mixtures used were not hypoxic.

### Statistical Analysis

Data are given as the mean ± the standard error to the mean. The effects of gas mixtures containing xenon and argon were analyzed with Statview software (SAS Institute, Cary, NC) and compared to those of control experiments, and xenon and argon alone, using non-parametric Mann-Whitney U-test.

### RESULTS

First, the neuroprotective effects of gas mixtures containing xenon and argon at equimolar concentrations of 15% to 37.5% were investigated in brain slices exposed to OGD, a model of brain ischemia, and in rats that were administered an intracerebral injection of NMDA.

OGD induced an increase in LDH release compared to control slices (P < 0.0001). As illustrated in Fig. 1, we found Xe-Ar-15 > Xe-Ar-25 > Xe-Ar-37.5 at reducing the release of LDH, a marker of cell injury, in brain slices exposed to OGD. Xe-Ar-15 decreased LDH (P < 0.0001 vs Air-treated OGD slices) in a manner similar to that of xenon at 50%. This led to a significant difference between Xe-Ar-15 and xenon at 15 vol% (P < 0.0001), which had no effect by itself on OGD-induced LDH release, and Xe-Ar-15 and argon at 15 vol% (P < 0.0001), which also had no effect by itself on OGD-induced LDH release. Xe-Ar-25 decreased OGD-induced LDH release (P < 0.0001) with similar amplitude than xenon alone at 25-37.5 vol%. This led to a significant difference between Xe-Ar-25 and argon at 25 vol% (P < 0.0001), which had no effect by itself on OGD-induced LDH release, but not with xenon at 25 vol%, thereby indicating that the neuroprotective effect of Xe-Ar-25 mainly resulted from the presence of xenon in the gas mixture. In contrast with Xe-Ar-15 and Xe-Ar-25, we found that Xe-Ar-37.5 failed to decrease LDH release in OGD slices.

Alternatively, intracerebral injection of NMDA in rats treated with air induced brain damage compared to air-treated controls injected with saline (P < 0.0001). As shown in Fig. 2, it was found Xe-Ar-15 > Xe-Ar-25 > Xe-Ar-37.5 at reducing brain damage in rats injected with NMDA. Xe-Ar-15 reduced NMDA-induced brain damage (P < 0.001) in a manner similar to that of xenon alone at 50 vol%. This led to a significant difference between Xe-Ar-15 and xenon at 15 vol% (P < 0.002), which had no effect by itself on NMDA-induced brain damage, and Xe-Ar-15 and argon at 15 vol% (P < 0.02), which also had no effect by itself on NMDA-induced brain damage. Likewise, Xe-Ar-25 reduced NMDA-induced brain damage (P < 0.02) with similar amplitude than xenon alone at 25-37.5 vol%. This led to a significant difference between Xe-Ar-25 and argon at 25 vol% (P < 0.05), which had no effect by itself on NMDA-induced brain damage, but not with xenon at 25 vol%, thereby indicating that the neuroprotective effect of Xe-Ar-25 mainly resulted from the presence of xenon in the gas mixture. In contrast with Xe-Ar-15 and Xe-Ar-25, Xe-Ar-37.5 failed to show neuroprotection in rats injected with intracerebral NMDA.

Next, because previous data have demonstrated that xenon and argon both interact with tPA [10,11], the effects of gas mixtures containing xenon and argon at equimolar concentration of 15 vol% to 37.5 vol% on the catalytic activity of tPA were studied. As illustrated in Fig. 3, it was found Xe-Ar-37.5 > Xe-Ar-25 > Xe-Ar-15 at reducing the catalytic efficiency of tPA. Xe-Ar-37.5 decreased the catalytic efficiency of tPA (P < 0.0001) to a similar extent than xenon alone at 75 vol%. This led to a significant difference between Xe-Ar-37.5 and xenon at 37.5 vol% (P < 0.0001), and Xe-Ar-37.5 and argon at 37.5 vol% (P < 0.0001). Likewise, Xe-Ar-25 also decreased the catalytic efficiency of tPA (P < 0.0001) to a similar extent than xenon alone at 50 vol%. This led to a significant difference between Xe-Ar-25 and xenon at 25 vol% (P < 0.0001) and between Xe-Ar-25 and argon at 25 vol% (P < 0.002). While xenon alone at 25 vol% had no effect on the catalytic efficiency of tPA, argon at 25 vol% decreased it significantly, thereby indicating that the reducing effect of Xe-Ar-25 on the catalytic efficiency of tPA mainly resulted from the effect of argon at 25 vol%. In contrast, we found that Xe-Ar-15 had no effect on the catalytic efficiency of tPA.

### DISCUSSION

In the present study, it was shown that gas mixtures containing low concentrations of xenon and argon of 15 vol% (Xe-Ar-15) or 25 vol% (Xe-Ar-25) of each gas can provide neuroprotection. Particularly, it was found that gas mixtures containing Xe-Ar-15 reduced OGD-induced cell injury and NMDA-induced brain damage to a similar extent than xenon alone at 50 vol%, thereby indicating that Xe-Ar-15 has potent neuroprotective effects. These potent neuroprotective effects of Xe-Ar-15, together with the fact that neither xenon alone at 15 vol% nor argon alone at 15 vol% (data not shown) had neuroprotective properties, is believed to be due to a synergism between inhibition by xenon of the NMDA receptor [8], which is the main excitatory receptor in the brain, and activation by argon of the GABA-A receptor [9], which is the main inhibitory receptor in the brain. Because Xe-Ar-15 is as potent as xenon alone at 50 vol%, and much more potent than argon alone at any concentration, at providing neuroprotection, it is likely that the mechanisms by which Xe-Ar-15 acts is mainly mediated through the NMDA receptor, which is the main neuronal target of xenon [10]. In contrast with the potent neuroprotective effects of Xe-Ar-15, it was found that Xe-Ar-37.5 failed to provide neuroprotection. The more plausible cause for this lack of effect of Xe-Ar-37.5 is a too potent general reduction in neuronal activity, which could be equivalent to narcosis or anesthesia in vivo. Support for this is the fact that neuroprotection decreased as a function of the concentration of Xe-Ar used (Xe-Ar-15 > Xe-Ar-25 > Xe-Ar-37.5). Further support for this are previous data that have shown that xenon provided neuroprotection at subanesthetic concentrations of 37.5 vol% or 50 vol% but not at high anesthetic concentrations of or above 75 vol% [5,6].

Alternatively, it was further shown that gas mixtures containing Xe-Ar-37.5 and Xe-Ar-25, but not Xe-Ar-15, reduced the catalytic efficiency of tPA to a similar extent than xenon at high concentrations of 75 vol% and 50 vol%, thereby suggesting the presence of another synergistic mechanism between xenon and argon at the tPA level [10,11]. Interestingly, structural biophysical studies have shown that both xenon and argon bind to the active site of elastase, a serine protease used as a model of tPA [12]. This is believed to explain the synergistic effect of Xe-Ar-25 and Xe-Ar-37.5 at inhibiting tPA-induced catalytic activity, which could be mainly mediated through xenon since argon alone has no effect of tPA-induced catalytic activity and further bind to elastase with a lower affinity than xenon [12].

Previous data in a rat model of thromboembolic stroke have shown that intraischemic xenon dose-dependently inhibits tPA-induced thrombolysis and subsequent reduction of ischemic brain damage [10], thereby leading to the conclusion that xenon should only be administered after, but not before or together, tPA-induced thrombolysis. In order to not favor reocclusion, a phenomenon shown to occur in 10 % to 15 % of ischemic stroke patients 2-3 h after tPA-induced reperfusion [13], it was further suggested that the use of xenon could require to be delayed according to a benefit-risk medical evaluation for the patient. Although, such a delay should not hamper dramatically the neuroprotective potential of xenon, suggested to have a possible therapeutic window of about 8 h [6], there is a general consensus that time is critical for treating ischemic stroke. Therefore, because as shown in the present study Xe-Ar-15 has no interaction with tPA and is as potent as xenon at 50 vol% at providing neuroprotection, Xe-Ar-15 is a promising alternative, cost-efficient, neuroprotective strategy to xenon alone for treating acute ischemic stroke.

### REFERENCES

1. Ryang YM, Fahlenkamp AV, Rossaint R, Loetscher PD, Beyer C, et al. Neuroprotective effect of argon in an in vivo model of transient middle cerebral artery occlusion in rats. Crit Care Med 2011; 39:1448-1453.
2. Jawad N, Rizvi M, Gu J, Adeyi O, Tao G, Maze M, Ma D. Neuroprotection (and lack of neuroprotection) afforded by a series of noble gases in an in vitro model of neuronal injury. Neurosci Lett 2009; 460:232-236.
3. David HN, Haelewyn B, Degoulet M, Colomb DG Jr, Risso JJ, Abraini JH. Ex vivo and in vivo neuroprotection induced by argon when given after an excitotoxic or ischemic insult. PLoS One 2012; 7:e30934.
4. Homi HM, Yokoo D, Ma D, Warner DS, Franks NP, Maze M, Grocott HP. The neuroprotective effect of xenon administration during transient middle cerebral artery occlusion in mice. Anesthesiology 2003; 99: 876-881.
5. David HN, Léveillé F, Chalzaviel L, MacKenzie ET, Buisson A, Lemaire M, Abraini JH. Reduction of ischemic brain damage by nitrous oxide and xenon. J Cereb Blood Flow Metab 2003; 23:1168-1173.
6. David HN, Haelewyn B, Rouillon C, Lecocq M, Chazalviel L, Apiou G, Risso JJ, Lemaire M, Abraini JH. Neuroprotective effects of xenon: a therapeutic window of opportunity in rats subjected to transient cerebral ischemia. FASEB J 2008; 22:1275-1286.
7. Dingley J, Tooley J, Porter H, Thoresen M. Xenon provides short-term neuroprotection in neonatal rats when administered after hypoxia-ischemia. Stroke 2006; 37, 501-506
8. Franks NP, Dickinson R, de Sousa SLM, Hall AC, Lieb WR. How does xenon produce anesthesia? Nature 1998; 396:324
9. Abraini JH, Kriem B, Balon N, Rostain JC, Risso JJ. GABA neuropharmacological investigations of narcosis produced by nitrogen, argon, and nitrous oxide. Anesth Analg 2003, 96:746-749.
10. David HN, Haelewyn B, Colloc'h N, Risso JJ, Abraini JH. Xenon is an inhibitor of tissue-plasminogen activator: adverse and beneficial effects in a rat model of thrombo-embolic stroke. J Cereb Blood Flow Metab 2010 30:718-728.
11. David HN, Haelewyn B, Risso JJ, Abraini JH. Modulation by the noble gas argon of the catalytic and thrombolytic efficiency of tissue plasminogen activator. Naunyn Schmiedebergs Arch Pharmacol 2013; 386:91-95.
12. Colloc'h N, Marassio G, Prangé T. Protein-Noble Gas Interactions Investigated by Crystallography on Three Enzymes - Implication on Anesthesia and Neuroprotection Mechanisms. Current Trends in X-Ray Crystallography, Dr. Annamalai Chandrasekaran (Ed.), ISBN: 978-953-307-754-3, InTech. Available on: http://cdn.intechopen.com/pdfs-wm/25143.pdf
13. Rubiera M, Alvarez-Sabin J, Ribo M, Montaner J, Santamarina E, Arenillas JF, Huertas R, Delgado P, Purroy F, Molina CA. Predictors of early arterial reocclusion after tissue plasminogen activator-induced recanalization in acute ischemic stroke. Stroke 2005; 36:1452-1456.
14. Dirnagl et al., Pathobiology of ischaemic stroke: an integrated view, Trends Neurosci. 22: 391-7, 1999.
15. Choi et al., Pharmacology of glutamate neurotoxicity in cortical cell culture: attenuation by NMDA antagonists. J Neurosci. 1988 Jan;8(1):185-96.
16. Sattler et al., Distinct Influx Pathways, Not Calcium Load, Determine Neuronal Vulnerability to Calcium Neurotoxicity, J. Neurochem., 71: (6), 2349-2364. 12 1998
17. Tsirka et al., Excitotoxin-induced neuronal degeneration and seizure are mediated by tissue plasminogen activator, Nature, 377: 340-344, 1995;
18. Wang et al., Tissue plasminogen activator (tPA) increase neuronal damage after focal cerebral ischemia in wild-type and tPA-deficient mice, Nature Med., 4: 228-231, 1998;

## Claims

1. A pharmaceutical composition comprising a gas mixture of xenon and argon for use in a method for preventing and/or treating ischemic insults, wherein
- the volume proportion of xenon is between 5 and 20 %,
- the volume proportion of argon is between 5 and 20 %, and
- the pharmaceutical composition further comprises a gas complement to reach 100% volume proportion,
- the method further includes restoration of blood flow and administering said pharmaceutical composition to the patient before, during, or after blood flow restoration.

2. The pharmaceutical composition for use of claim 1, wherein the ischemic insult is cerebral ischemia, cardiac ischemia, renal ischemia, retinal ischemia, or lower limb's ischemia.

3. The pharmaceutical composition for use of claim 1 or 2, wherein blood flow is restored spontaneously, or medically by thrombolysis induction and/or by thrombectomy.

4. The pharmaceutical composition for use of claim 3, wherein thrombolysis is induced by administering a pharmaceutical agent with thrombolytic properties.

5. The pharmaceutical composition for use of claim 4, wherein the pharmaceutical agent with thrombolytic properties is a thrombolytic agent such as streptokinase, urokinase, alteplase (human recombinant tissue-type plasminogen activator or rtPA), reteplase or tenecteplase.

6. The pharmaceutical composition for use of claim 1, wherein the ischemic insult is caused by a decompression accident, and blood flow is restored by recompression.

7. The pharmaceutical composition for use of any of claims 1 to 6, wherein
- the volume proportion of xenon is between 10 and 20 %, and
- the volume proportion of argon is between 10 and 20 %.

8. The pharmaceutical composition for use of any of claims 1 to 7, wherein the xenon/argon volume ratio is between 4/1 and 1/4, preferably 1/1.

9. The pharmaceutical composition for use of any of claims 1-8, wherein the volume proportion of xenon is 15%, and the volume proportion of argon is 15 %.

10. The pharmaceutical composition for use of any of claims 1 to 9, wherein the gas complement comprises oxygen, nitrogen, helium, neon, or a mixture thereof.

11. The pharmaceutical composition for use of any of claims 1 to 10, wherein the gas complement comprises oxygen in a volume proportion of between 20 % and 50 %, and wherein the remainder of the gas in the gas complement is nitrogen, helium, hydrogen, neon, or a mixture thereof, preferably helium.

12. The pharmaceutical composition of any of claims 1-7, wherein the pharmaceutical composition is preconditioned as a compressed gas mixture.

13. The pharmaceutical composition of any of claims 1-7, wherein the pharmaceutical composition administered to the patient is obtained by mixing argon, xenon and the gas complement in a gas mixer.
